# EUROPÄISCHE PATENTSCHRIFT

(11) **EP 1 729 690 B1**
(45) Veröffentlichungstag und Bekanntmachung des Hinweises auf die Patenterteilung: **03.02.2010**
(21) Anmeldenummer: 04725264.8
(22) Anmeldetag: 02.04.2004
(51) Int. Cl.: A61F 2/44

(54) **MODULARES ZWISCHENWIRBELIMPLANTAT ODER BANDSCHEIBENPROTHESE**
MODULAR INTERVERTEBRAL IMPLANT OR INTERVERTEBRAL DISC PROSTHESIS
IMPLANT INTERVERTEBRAL OU PROTHESE DE DISQUE INTERVERTEBRAL MODULAIRE

(43) Veröffentlichungstag der Anmeldung: 13.12.2006
(73) Patentinhaber: Synthes GmbH, 4436 Oberdorf (CH)
(72) Erfinder: STUDER, Armin, CH-6330 Cham (CH)
(74) Vertreter: Lusuardi, Werther
(86) Internationale Anmeldenummer: PCT/CH2004/000209
(87) Internationale Veröffentlichungsnummer: WO 2005/094732

(56) Entgegenhaltungen:
- EP-A- 0 566 810
- EP-A- 1 374 807
- US-A- 5 360 430
- US-A1- 2003 014 111
- US-A1- 2003 187 506
- US-B1- 6 375 682

## Beschreibung

Die Erfindung bezieht sich auf ein modulares Zwischenwirbelimplantat oder eine modulare Bandscheibenprothese mit verschiedenen Mittelstücken und einer Anzahl daran lösbar befestigbarer Appositionsplatten unterschiedlicher Bauweise.

Dies hat zur Folge, dass in Abhängigkeit vom betroffenen Wirbelsäulensegment, vom Gewicht des Patienten und vom Anwendungsfall ohne grosse Anzahl unterschiedlicher implantate dem Chirurgen bereit gestellt werden müssen, aus der dieser dann seine Wahl treffen kann.

Der nächstliegende Stand der Technik ist z.B. in EP-A-0 566 810 beschrieben.

Hier will die Erfindung Abhilfe schaffen. Der Erfindung liegt die Aufgabe zugrunde, ein modulares Zwischenwirbelimplantat oder eine modulare Bandscheibenprothese zu schaffen, welche in Form eines Baukastens aus einer Anzahl Mittelstücken (für unterschiedlich schwere Patienten sowie für Patienten mit unterschiedlichem Bewegungsumfang) und einer Anzahl daran lösbar befestigbarer Appositionsplatten unterschiedlicher Bauart vorliegt, so dass der Chirurg, das von ihm als optimal erachtete implantat prä-operativ selbst zusammenstellen kann und falls notwendig auch noch intraoperativ an die anatomischen Gegebenheiten und Erfordernisse durch Austausch geeigneter Mittelteile oder Appositionsplatten anpassen kann.

Die Erfindung löst die gestellte Aufgabe mit einem Mittelteil, welches die Merkmale des Anspruchs 1 aufweist, einem modularen Zwischenwirbelimplantat oder Bandscheibenprothese, welche die Merkmal des Anspruchs 9 aufweist, sowie einem modularen Baukasten, welcher die Merkmale des Anspruchs 14 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass sie einen einfachen Ausgleich bei anatomisch unterschiedlichen Wirbeln zulässt, wie z.B. bei der Kombination Sakrum 1 und Lumbaler Wirbel Nr. 5 (S1/L5).

Das Mittelteil ist parallel zu seiner Längsachse elastisch deformierbar und weist eine progressive Federkennlinie f auf. Durch die progressive Federkennlinie f wird erreicht, dass das Mittelstück für Belastungen von verschieden schweren Patienten einsetzbar ist. Die Progressivität der Federkennlinie f mindestens eines Mittelstücks ist erfindungsgemäss derart ausgebildet, dass:
- ein erster Teilbereich für einen Federweg zwischen 0 mm und 0,5 mm mit einer Federrate zwischen 450 N/mm und 550 N/mm, vorzugsweise ca. 500 N/mm vorhanden ist, so dass das Mittelstück für Belastungen eines 40 - 65 kg schweren Patienten geeignet ist; und
- ein zweiter Teilbereich für den Federweg 0,5 mm bis 1,0 mm mit einer Federrate zwischen 1400 N/mm und 1600 N/mm, vorzugsweise von ca. 1500 N/mm vorhanden ist, so dass das Mittelstück für Belastungen eines 60 - 100 kg schweren Patienten geeignet ist.

In einer bevorzugten Ausführungsform sind das obere und das untere Ende des Mittelstückes mit den Mitteln zur lösbaren Befestigung an je einer Appositionsplatte versehen. Dadurch können an beiden Enden das Mittelstückes verschiedene Appositionsplatten angebracht werden, was eine individuelle Anpassung des aus Mittelteil und Endplatten zusammengefügten Zwischenwirbelimplantates ermöglicht.

Vorzugsweise sind die Mittel zur lösbaren Befestigung des Mittelteils an den Appositionsplatten parallel zur Längsachse in Eingriff bringbar, so dass ein einfaches Zusammenfügen des Zwischenwirbelimplantates erreichbar ist.

In einer anderen Ausführungsform sind die Mittel durch eine zur Längsachse konzentrische Rotation des Mittelstückes relativ zu einer Appositionsplatte verriegelbar, wobei die Mittel vorzugsweise in Form eines weiblichen oder männlicher Teils eines Bajonettverschlusses realisiert sind. Der Bajonettverschluss kann als Drehverschluss ausgebildet sein, so dass eine einfache Bedienbarkeit erreichbar ist.

In wiederum einer anderen Ausführungsform ist die Progressivität der Federkennlinie f derart ausgebildet, dass:
- ein dritter Teilbereich für den Federweg 1,0 mm bis 1,5 mm mit einer Federrate zwischen 2500 N/mm und 3500 N/mm, vorzugsweise von ca. 3000 N/mm vorhanden ist, so dass das Mittelstück für Belastungen eines 100 - 140 kg schweren Patienten geeignet ist.

In einer weiteren Ausführungsform umfasst das Mittelstück axial endständig je eine quer zur Längsachse angeordnete Endplatte, welche axial aussenstehend je den weiblichen Teil des Bajonettverschlusses aufweisen. Der komplementär ausgebildete männliche Teil des Bajonettverschlusses müsste dann an den gegen das Mittelstück gerichteten Oberflächen der Appositionsplatten angebracht sein.

In einer bevorzugten Ausführungsform der erfindungsgemässen Appositionsplatte umfasst diese Mittel zur lösbaren Befestigung an einer der oben aufgeführten Ausführungsformen des Mittelstückes und weist eine zur Apposition an die Grund- oder Deckplatte eines Wirbelkörpers geeignete äussere Oberfläche und eine innere Oberfläche mit den Mitteln auf. Vorzugsweise ist die äussere Oberfläche konvex nach aussen gewölbt ist.

In einer anderen Ausführungsform schliesst die äussere Oberfläche der Appositionsplatte relativ zur inneren Oberfläche einen spitzen Winkel α ein, welcher vorzugsweise im Bereich von 2° bis 7° liegt.

In wiederum einer anderen Ausführungsform weist die äussere Oberfläche der Appositionsplatte eine dreidimensionale Strukturierung auf.

Das erfindungsgemässe modulares Zwischenwirbelimplantat oder modulare Bandscheibenprothese umfasst vorzugsweise ein Mittelstück gemäss einer der oben beschriebenen Ausführungsformen und zwei Appositionsplatten gemäss einer der oben beschriebenen Ausführungsformen.

Appositionsplatten verwendet werden, wobei der modulare Baukasten n ≥ 2 Mittelstücke und m ≥ 4 Appositionsplatten, vorzugsweise n ≥ 3 Mittelstücke und m ≥ 6 Appositionsplatten umfasst. Der modulare Baukasten ermöglicht ein optimales Anpassen des zu implantierenden Zwischenwirbelimplantates oder der zu implantierenden Bandscheibenprothese und ermöglicht ein Zusammenstellen des Zwischenwirbelimplantates in-situ.

Vorzugsweise weisen mindestens zwei der m Appositionsplatten eine unterschiedliche Höhe auf.

Die Erfindung und Weiterbildungen der Erfindung werden im Folgenden anhand der teilweise schematischen Darstellungen mehrerer Ausführungsbeispiele noch näher erläutert.

Es zeigen:
Fig. 1 einen Schnitt durch eine Ausführungsform des erfindungsgemässen Mittelteils;
Fig. 2 einen Schnitt entlang der Linie A - A in Fig. 1;
Fig. 3 die Ausführungsform einer Appositionsplatte, welche oberhalb des Mittelteils an diesem befestigbar ist;
Fig. 4 die in Fig. 3 dargestellte Ausführungsform der Appositionsplatte, welche unterhalb des Mittelteils an diesem befestigbar ist;
Fig. 5 eine Aufsicht auf die in den Fig. 1 und 2 dargestellte Ausführungsform des erfindungsgemässen Mittelteils; und
Fig. 6 einen antero-posterioren Schnitt durch eine Ausführungsform einer modularen Bandscheibenprothese.

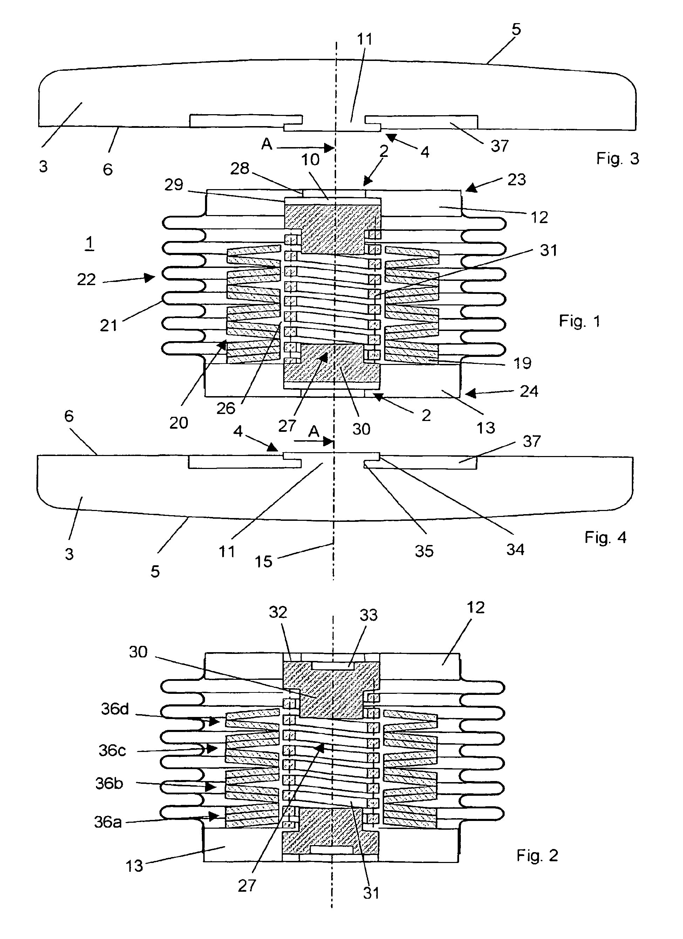

In den Fig. 1, 2 und 5 ist eine Ausführungsform des Mittelteils 1 dargestellt, welches im wesentlichen ein erstes Ende 23, ein zweites Ende 24, ein zur Längsachse 15 koaxial angeordnetes, hohlzylindrisches elastisches Mittel 20, einen als Faltenbalg 21 ausgebildeten koaxialen Mantel 22, je eine axial endständig quer zur Längsachse 15 angeordnete Endplatte 12;13 und im Hohlraum 26 des elastischen Mittels 20 angeordnete axial elastische Arretiermittel 27 zur Arretierung der in den Endplatten 12;13 angeordneten Bajonettverschlüsse.

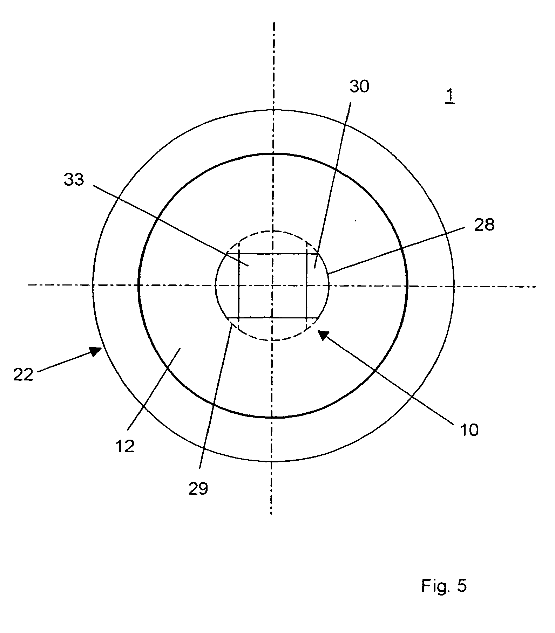

Die Endplatten 12;13 umfassen endständig das weibliche Teil 10 der Bajonettverschlüsse in Form einer aussenstehenden ovalen Öffnung 28 (Fig. 5) und einem axial angrenzenden zylindrischen Hinterstich 29, so dass die an den Appositionsplatten 3 (Fig. 3 und 4) angeordneten, komplementären männlichen Teile 11 der Bajonettverschlüsse einführbar und durch Drehung um die Längsachse 15 axial fixierbar sind. Zur Arretierung der männlichen Teile 11 der Bajonettverschlüsse dienen die Arretiermittel 27, welche hier zwei axial übereinander angeordnete Zapfen 30 und eine dazwischen koaxial angeordnete Feder 31 umfassen. Die Zapfen 30 werden durch die Feder 30 gegen die Endplatten 12;13 gepresst. Die Zapfen 30 weisen an ihren axial aussenstehenden Enden 32 je eine quer zur Längsachse 15 angeordnete Nute 33 (Fig. 5) zur Aufnahme der vorderen Segmente 34 der männlichen Teile 11 (Fig. 3 und 4) der Bajonettverschlüsse auf.

Die männlichen Teile 11 der Bajonettverschlüsse sind in den Fig. 3 und 4 ersichtlich. Sie umfassen je ein vorderes Segment 34, welches komplementär zur ovalen Öffnung 28 in den weiblichen Teilen 10 der Bajonettverschlüsse ausgebildet ist, und ein hinteres Segment 35, welches zylindrisch ausgebildet ist und das vordere Segment 34 radial nicht überragt, so dass die männlichen Teile 11 nach dem Einführen in die weiblichen Teile 10 um die Längsachse 15 drehbar sind, bis die vorderen Segmente 33 in die Nuten 33 an den Zapfen 30 einschnappen. Während des Einführens werden dazu die Zapfen 30 axial gegeneinander verschoben, so dass nach der Drehung der männlichen Teile 11 der Bajonettverschlüsse, wenn die vorderen Segmente 34 mit den Nuten 33 fluchten, die Zapfen 30 durch die Federkraft der Feder 31 gegen die Endplatten 12;13 verschoben werden und die vorderen Segmente 34 der männlichen Teile 11 in den Nuten 33 blockiert sind.

Das Mittelteil 1 umfasst ferner zur Längsachse 15 koaxial angeordnete elastische Mittel 20 und aussen um die elastischen Mittel 20 einen koaxialen, als Faltenbalg 21 ausgebildeten Mantel 22. Je nach Material kann der Faltenbalg 21 an den zwei Endplatten 12;13 des Mittelteils 1 angeschweisst, verstemmt oder eingepresst sein. In der hier dargestellten Ausführungsform sind die elastischen Mittel 20 aus Tellerfederpaketen 36a;36b;36c;36d (Fig. 2) mit gleichen Tellerfedern 19 zusammengesetzt, wobei jedoch das erste Tellerfederpaket 36a aus einer drei gleichgerichtet geschichtete Tellerfedern 19 umfassenden Tellefedergruppe besteht, das zweite und dritte Tellerfederpaket 36b;36c je zwei gegeneinander gerichtete Gruppen aus je zwei gleichgerichtet geschichteten Tellerfedern 19 besteht und das vierte Tellerfederpaket 36d aus zwei gegeneinander gerichteten Tellerfedern 19 besteht. Durch diese Ausgestaltung der Tellerfederpakete 36a;36b;36c; 36d ist eine progressive Federkennlinie der elastischen Mittel 20 erreichbar.

In den Fig. 3 und 4 dargestellt sind eine am oberen Ende 23 des Mittelteils 1 quer zur Längsachse 15 angeordnete, obere Appositionsplatte 3, welche zur Anlage an die Grundplatte eines Wirbelkörpers geeignet ist, und eine am unteren Ende 24 des Mittelteils 1 quer zur Längsachse 15 angeordnete, untere Appositionsplatte 3 welche zur Anlage an die Deckplatte eines Wirbelkörpers geeignet ist. Die beiden Appositionsplatten 3 haben gegen aussen eine konvex gewölbte Oberfläche 5 und weisen an ihren inneren Oberflächen 6 eine zur Längsachse 15 konzentrische Vertiefung 37 mit dem integrierten männlichen Teil 11 des Bajonettverschlusses auf.

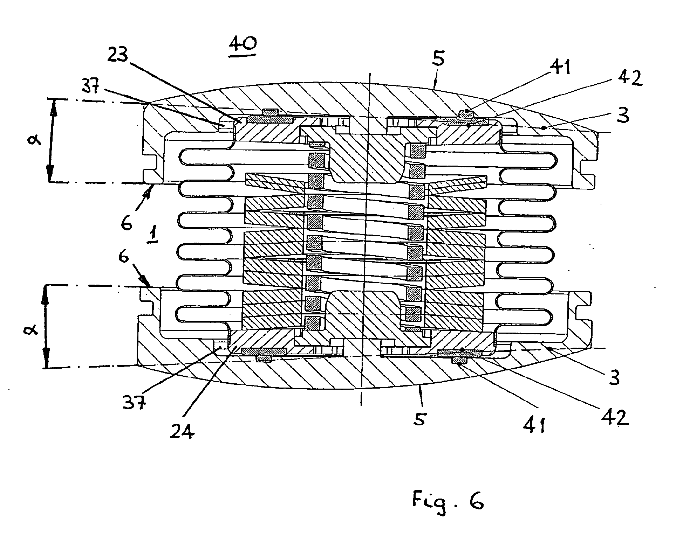

Fig. 6 zeigt eine Ausführungsform des modularen Zwischenwirbelimplantates 40 mit einem Mittelteil 1, einer oberen Appositionsplatte 3 am oberen Ende 23 des Mittelteils 1 und einer unteren Appositionsplatte 3 am unteren Ende 24 des Mittelteils 1. Das Mittelteil 1 und die Appositionsplatten 3 unterscheiden sich nur darin von den in den Fig. 1 bis 5 dargestellten Ausführungsformen, dass in den Vertiefungen 37 an den inneren Oberflächen 6 der Appositionsplatten 3 und komplementär aussen an den Endplatten 12;13 des Mittelteils 1 je ein zur Längsachse 15 konzentrischer Ring 41;42 aus einem keramischen Material angeordnet ist, und dass die äusseren Oberflächen 5 der Appositionsplatten 3 einen Winkel α von 3° mit den inneren Oberflächen 6 einschliessen. Diese Abschrägung der Appositionsplatten 3 ist derart ausgebildet, dass die Gesamthöhe des Zwischenwirbelimplantates 40 dorsal kleiner ist als ventral. Die keramischen Ringe 41;42 übernehmen eine Dichtungsfunktion, so dass keine Körperflüssigkeit ins Innere des Zwischenwirbelimplantates eindringen kann.

## Patentansprüche

1. Mittelstück (1) für ein modulares Zwischenwirbelimplantat oder eine modulare Bandscheibenprothese, welches mit Mitteln (2) zur lösbaren Befestigung an einer zur Apposition an die Grund- oder Deckplatte eines Wirbelkörpers geeigneten Appositionsplatte (3) versehen ist, wobei
das Mittelstück (1) eine Längsachse (15) aufweist, parallel dazu elastisch ist und eine progressive Federkennlinie f aufweist; **dadurch gekennzeichnet, daß**
die Federkennlinie f in einem ersten Teilbereich für einen Federweg zwischen 0 mm und 0,5 mm eine Federrate zwischen 450 N/mm und 550 N/mm und in einem zweiten Teilbereich für einen Federweg 0,5 mm bis 1,0 mm eine Federrate zwischen 1400 N/mm und 1600 N/mm aufweist.

2. Mittelstück (1) nach Anspruch 1, **dadurch gekennzeichnet, dass** es ein oberes und ein unteres Ende (23;24) aufweist und an beiden Enden (23;24) mit Mitteln (2) zur lösbaren Befestigung an einer Appositionsplatte (3) versehen ist.

3. Mittelstück (1) nach Anspruch 1 oder 2, **dadurch gekennzeichnet, dass** es eine das obere und das untere Ende (23;24) schneidende Längsachse (15) aufweist, und dass die Mittel (2) parallel zur Längsachse (15) an einer Appositionsplatte (3) in Eingriff bringbar sind.

4. Mittelstück (1) nach Anspruch 3, **dadurch gekennzeichnet, dass** die Mittel (2) durch zur Längsachse (15) konzentrische Rotation des Mittelstückes (1) relativ zu einer Appositionsplatte (3) verriegelbar sind.

5. Mittelstück (1) nach einem der Ansprüche 1 bis 4, **dadurch gekennzeichnet, dass** die Federkennlinie f in einem Teilbereich eine Federrate zwischen 2500 N/mm und 3500 N/mm aufweist.

6. Mittelstück (1) nach einem der Ansprüche 1 bis 5, **dadurch gekennzeichnet, dass** die Mittel (2) in Form eines weiblichen oder männlichen Teils (10;11) eines Bajonettverschlusses realisiert sind.

7. Mittelteil (1) nach Anspruch 6, **dadurch gekennzeichnet, dass** der Bajonettverschluss als Drehverschluss ausgebildet ist.

8. Mittelstück (1) nach Anspruch 6 oder 7, **dadurch gekennzeichnet, dass** das Mittelstück (1) axial endständig je eine quer zur Längsachse (10) angeordnete Endplatte (11;12) umfasst, welche axial aussenstehend je den weiblichen Teil (10) des Bajonettverschlusses aufweist.

9. Modulares Zwischenwirbelimplantat (40) oder modulare Bandscheibenprothese mit einem Mittelstück (1) nach einem der Ansprüche 1 bis 8 und zwei Appositionsplatten (3) mit Mitteln (4) zur lösbaren Befestigung am Mittelstück (1), **dadurch gekennzeichnet, dass** die Mittel (4) mit den Mitteln (2) des Mittelstücks (1) korrespondieren.

10. Modulares Zwischenwirbelimplantat (40) oder modulare Bandscheibenprothese nach Anspruch 9, **dadurch gekennzeichnet, dass** die Appositionsplatten (3) eine zur Apposition an die Grund- oder Deckplatte eines Wirbelkörpers geeignete äussere Oberfläche (5) und eine innere Oberfläche (6) mit den Mitteln (4) aufweisen.

11. Modulares Zwischenwirbelimplantat (40) oder modulare Bandscheibenprothese nach Anspruch 10, **dadurch gekennzeichnet, dass** die äussere Oberfläche (5) konvex nach aussen gewölbt ist.

12. Modulares Zwischenwirbelimplantat (40) oder modulare Bandscheibenprothese nach Anspruch 10 oder 11, **dadurch gekennzeichnet, dass** die äussere Oberfläche (5) relativ zur inneren Oberfläche (6) einen spitzen Winkel α einschliesst, vorzugsweise im Bereich von 2° bis 7°.

13. Modulares Zwischenwirbelimplantat (40) oder modulare Bandscheibenprothese nach einem der Ansprüche 10 bis 12, **dadurch gekennzeichnet, dass** die äussere Oberfläche (5) eine dreidimensionale Strukturierung aufweist.

14. Modularer Baukasten mit n Mittelstücken (1) nach einem der Ansprüche 1 bis 8 und m Appositionsplatten (3), **dadurch gekennzeichnet, dass** n ≥ 2 und m ≥ 4 ist, vorzugsweise dass n ≥ 3 und m ≥ 6 ist.

15. Modularer Baukasten nach Anspruch 14, **dadurch gekennzeichnet, dass** mindestens zwei der m Appositionsplatten (3) eine unterschiedliche Höhe aufweisen.

## Claims

1. Centerpiece (1) for a modular intervertebral implant or a modular intervertebral disc prosthesis, fitted with means (2) for the detachable fixation to a juxtaposition plate (3) suitable for a juxtaposition to the base plate or cover plate of a vertebral body, wherein the centerpiece (1) has a longitudinal axis (15), is elastic parallel to the same and has a progressive spring characteristic f,
**characterized in that**
the spring characteristic f exhibits in a first partial region for a spring excursion between 0 mm and 0.5 mm a spring rate between 450 N/mm and 550 N/mm, and in a second partial region for a spring excursion between 0.5 mm and 1.0 mm a spring rate between 1400 N/mm and 1600 N/mm.

2. Centerpiece (1) according to claim 1, **characterized in that** it has an upper and a lower extremity (23;24) and is at both extremities (23;24) fitted with means (2) for a detachable fixation to a juxtaposition plate (3).

3. Centerpiece (1) according to claim 1 or 2, **characterized in that** it has a longitudinal axis (15) intersecting the upper and the lower extremity (23;24) and that the means (2) can be engaged with a juxtaposition plate (3) parallel to the longitudinal axis (15).

4. Centerpiece (1) according to claim 3, **characterized in that** the means (2) can be locked with respect to a juxtaposition plate (3) by rotating the centerpiece (1) concentrically to the longitudinal axis (15).

5. Centerpiece (1) according to one of the claims from 1 to 4, **characterized in that** the characteristic spring curve f exhibits in a partial region a spring coefficient between 2500 N/mm and 3500 N/mm.

6. Centerpiece (1) according to one of the claims from 1 to 5, **characterized in that** the means (2) are realized in the shape of a female or a male element (10;11) of a bayonet lock.

7. Centerpiece (1) according to claim 6, **characterized in that** the bayonet lock is conformed as a rotating lock.

8. Centerpiece (1) according to claim 6 or 7, **characterized in that** the centerpiece (1) comprises at each of the axial extremities a terminal plate (11;12) fitted at an axially external position with the respective female element (10) of the bayonet lock.

9. Modular intervertebral implant (40) or modular intervertebral disc prosthesis with a centerpiece (1) according to one of the claims from 1 to 8, and two juxtaposition plates (3) with means for a detachable fixation to the centerpiece (1), **characterized in that** the means (4) correspond to the means (2) of the centerpiece (1).

10. Modular intervertebral implant (40) or modular intervertebral disc prosthesis according to claim 9, **characterized in that** the juxtaposition plates (3) present an external surface (5) suitable for a juxtaposition to the base plate or the cover plate of a vertebral body, and an internal surface (6) fitted with the means (4).

11. Modular intervertebral implant (40) or modular intervertebral disc prosthesis according to claim 10, **characterized in that** the external surface (5) is curved to the outside in a convex manner.

12. Modular intervertebral implant (40) or modular intervertebral disc prosthesis according to claim 10 or 11, **characterized in that** the external surface (5) comprises a sharp angle α, preferably in a range from 2° to 7°, with respect to the internal surface (6).

13. Modular intervertebral implant (40) or modular intervertebral disc prosthesis according to the claims from 10 to 12, **characterized in that** the internal surface (5) presents a tri-dimensional structure.

14. Modular construction set with n centerpieces (1) according to one of the claims from 1 to 8 and m juxtaposition plates (3), **characterized in that** n≥2 and m≥4, preferably n ≥ and m≥ 6.

15. Modular construction set (40) according to claim 14, **characterized** that at least two of the m juxtaposition plates (3) present a different height.

## Revendications

1. Élément central (1) pour un implant intervertébral modulaire ou une prothèse de disque intervertébral modulaire, pourvu de moyens (2) permettant la fixation amovible à une plaque d'apposition (3) appropriée à l'apposition sur le plateau de base ou le plateau supérieur d'un corps vertébral, dans lequel :
l'élément central (1) présente un axe longitudinal (15), une certaine élasticité parallèlement à cet axe et une caractéristique de ressort f progressive;
**caractérisé en ce que** :
la caractéristique de ressort f, pour une course de ressort comprise entre 0 mm et 0,5 mm, dans une première zone partielle, présente une constante de rappel comprise entre 450 N/mm et 550 N/mm et pour une course de ressort de 0,5 mm à 1,0 mm, dans une deuxième zone partielle, présente une constante de rappel comprise entre 1 400 N/mm et 1 600 N/mm.

2. Élément central (1) selon la revendication 1, **caractérisé en ce qu'**il présente une extrémité supérieure et une extrémité inférieure (23 ; 24) et qu'il est pourvu, au niveau des deux extrémités (23 ; 24), de moyens (2) permettant la fixation amovible à une plaque d'apposition (3).

3. Élément central (1) selon la revendication 1 ou 2, **caractérisé en ce qu'**il présente un axe longitudinal (15) qui coupe l'extrémité supérieure et l'extrémité inférieure (23 ; 24), et **en ce que** les moyens (2) peuvent venir en prise avec une plaque d'apposition (3) parallèlement à l'axe longitudinal (15).

4. Élément central (1) selon la revendication 3, **caractérisé en ce que** les moyens (2) peuvent être verrouillés au moyen d'une rotation concentrique à l'axe longitudinal (15) de l'élément central (1) par rapport à une plaque d'apposition (3).

5. Élément central (1) selon l'une quelconque des revendications 1 à 4, **caractérisé en ce que**, dans une zone partielle, la caractéristique de ressort f présente une constante de rappel comprise entre 2 500 N/mm et 3 500 N/mm.

6. Élément central (1) selon l'une quelconque des revendications 1 à 5, **caractérisé en ce que** les moyens (2) sont réalisés sous la forme d'un élément femelle ou d'un élément mâle (10 ; 11) d'une fermeture à baïonnette.

7. Élément central (1) selon la revendication 6, **caractérisé en ce que** la fermeture à baïonnette est réalisée en tant que verrou tournant.

8. Élément central (1) selon la revendication 6 ou 7, **caractérisé en ce que** l'élément central (1) comprend, à chaque extrémité axiale, un plateau d'extrémité (11 ; 12) disposé transversalement à l'axe longitudinal (10), lequel plateau d'extrémité présente, de chaque côté axial extérieur, l'élément femelle respectif (10) de la fermeture à baïonnette.

9. Implant intervertébral modulaire (40) ou prothèse de disque intervertébral modulaire comprenant un élément central (1) selon l'une quelconque des revendications 1 à 8 et deux plaques d'apposition (3) pourvues de moyens (4) permettant la fixation amovible à l'élément central (1), **caractérisé en ce que** les moyens (4) correspondent aux moyens (2) de l'élément central (1).

10. Implant intervertébral modulaire (40) ou prothèse de disque intervertébral modulaire selon la revendication 9, **caractérisé en ce que** les plaques d'apposition (3) présentent une surface externe (5) et une surface interne (6) comprenant les moyens (4), lesquelles surfaces sont appropriées à l'apposition sur le plateau de base ou le plateau supérieur d'un corps vertébral.

11. Implant intervertébral modulaire (40) ou prothèse de disque intervertébral modulaire selon la revendication 10, **caractérisé en ce que** la surface externe (5) est bombée de manière convexe vers l'extérieur.

12. Implant intervertébral modulaire (40) ou prothèse de disque intervertébral modulaire selon la revendication 10 ou 11, **caractérisé en ce que** la surface externe (5) forme un angle aigu α par rapport à la surface interne (6), de préférence dans la gamme de 2° à 7°.

13. Implant intervertébral modulaire (40) ou prothèse de disque intervertébral modulaire selon l'une quelconque des revendications 10 à 12, **caractérisé en ce que** la surface externe (5) présente une texture en trois dimensions.

14. Système modulaire comprenant n éléments centraux (1) selon l'une quelconque des revendications 1 à 8 et m plaques d'apposition (3), **caractérisé en ce que** n ≥ 2 et m ≥ 4, de préférence n ≥ 3 et m ≥ 6.

15. Système modulaire selon la revendication 14, **caractérisé en ce qu'**au moins deux des m plaques d'apposition (3) présentent des hauteurs différentes.
